# EUROPEAN PATENT APPLICATION

(11) **EP 3 922 729 A1**
(43) Date of publication of application: **15.12.2021**
(21) Application number: 20382510.4
(22) Date of filing: 12.06.2020
(51) Int. Cl.: C12Q 1/04, C12Q 1/24, C12Q 1/6888, C12M 1/26, C12M 1/12, G01N 33/497, G01N 1/22

(54) **DEVICE AND METHOD FOR CAPTURING AND ANALYSING AIRBORNE ORGANISMS**

(71) Applicant: Consejo Superior de Investigaciones Científicas (CSIC), 28006 Madrid (ES)
(72) Inventor: ALCAMI PERTEJO, Antonio, 28049 Madrid (ES); RASTROJO LASTRAS, Alberto, 28049 Madrid (ES)
(74) Representative: Pons

(57) **Abstract**

The present invention refers to a device comprising polytetrafluorethylene (PTFE) filters and the use of the same for collecting, detecting and identifying organisms present in air ecosystems. The invention also provides a method suitable for the capture, detection and identification of whole airborne biological particles, including viruses and other important air pathogens, which involves the use of the device. This method allows toperform organism, preferably viral, metagenomics to sequence all DNA and RNA organisms captured in the filters. This methodology may be used to detect, for instance, SARS-CoV2 particles in air samples.

## Description

The invention belongs to the fields of microbiology, public health and methods for analysing air ecosystems, in particular to methods and devices useful for collecting air samples and analysing the airborne microbiota present in them. These methods allow to gain a global view of the airborne biological communities as well as to identify pathogens (viruses, bacteria or fungi), allergens and the like present in them.

### BACKGROUND ART

Air pollution is known to be a major environmental risk to public health. In addition to inorganic pollution, air also carries several airborne biological particles (ABPs) containing archaea, bacteria, virus, fungi and pollen grains, coming from other environments (soil, water or plant/animals microenvironments). A great effort is being made by the international community to reduce air pollution but very little is known about the biological community (airbiota) present in the outdoor air. The study of the airbiota is relevant due to its potential role in dissemination of plant, animal and human diseases, with important implications in public health, and a huge economic impact on crops and stockbreeding productivity. The current knowledge about air microbial community is mainly restricted to pollen and fungal spores, studied using traditional methods such as optical microscopy or quantitative PCR, which because of its importance as allergens are daily measured and included among air quality indicators. Culture-dependent techniques were also used for the study of air microorganisms (fungi, bacteria and viruses), despite the small fraction of organisms that can be cultured. Furthermore, studies on viruses are almost inexistent. Therefore, a global overview of the airborne biological community is crucial to understand air ecosystem dynamics, to identify marker organisms and to establish new air quality indicators. However, there is not a standardized method to capture the whole diversity present in the air.

The interest in the study of airborne microbiota is increasing to understand the connectivity of different ecosystems, the survival of microbes in the air ecosystem and the transmission of human pathogens. In spite of its relevance, the characterization of microbiota in the air is in its infancy. A variety of sampling devices are being used by the research laboratories that have initiated these studies, but detailed studies on the performance of these devices are scarce.

Some of the airborne microorganisms are the causative agent of important human diseases such as *Influenza* virus, *Rhinoviruses, Mycobacterium tuberculosis* or *Aspergillus spp.* Others, although non-pathogenic, are known to act as allergens, such as grass pollen or *Alternaria* spores, affecting to several million people worldwide. Traditionally, air was considered mainly as a dissemination path for these microorganisms, being studied primary for its important implications in human health and livestock and agriculture productivity. However, in recent studies, the scientific community has suggested that air has its own biota, and thus, must be considered as an ecosystem in itself. Nevertheless, very little is known about airborne biological community, the dynamics governing them or the potential relations among microorganisms and the influence of environmental factors on its composition.

Next-generation sequencing (NGS) is a promising approach to study global microbial diversity that is being applied to other ecosystems such as ocean, freshwater bodies or thermal springs (Rastrojo A., Alcamí A., 2018, Adv Virus Res, 101:39-54; Rastrojo A., Alcamí A., 2017, Virus Res, 239:87-96). NGS was also applied to the study of air communities, mainly through targeted metagenomics of marker genes, but most of the studies were focused on a single group of organisms, mainly bacteria, or only on a couple of groups (fungi and bacteria) (Núñez A., et al., 2016, Int Microbiol, 19:1-13). Among airborne microorganisms, viruses have received little attention. Although there are many studies on specific viruses, such as *Influenza* virus, only one report has characterized the global viral diversity in outdoor air (Whon TW., et al., 2012, J Virol 86:8221-8231). Moreover, to date, only a few studies have used a shotgun sequencing approach to study whole airborne community, including viruses (Cao C., et al., 2014, Enviromental Science and Technology, 48:1499-1507; Be N. A. et al., 2015, Microb Ecol 69:346-355; Yooseph S., et al., 2013, PLoS One 8:e81862).

A number of devices with very different capture mechanisms have been used to study the airborne community (Núñez A., et al., 2016, Int Microbiol, 19:69-80). Therefore, the evaluation of different capturing methods is required to establish a standarized framework. It has been previously demonstrated that Hirst-type samplers are a good choice for monitoring the airborne biological community, by comparing classical approaches such as optical microscopy of pollen and fungal spores with targeted metagenomics, resulting in a good correlation between both techniques (Núñez A., et al., 2017, Appl Environ Microbiol, 83). Although Hirst samplers can give a good description of the airborne biological community, it is far from being complete. Viruses cannot be studied by targeted metagenomics using marker genes such as 16S/18S rRNA and others approaches are required. Hirst samplers are based on the inertial capture of ABPs in a vaseline covered strip that can be used for DNA extraction. A shotgun sequencing of this DNA could give a more complete view of the airborne community including DNA viruses. However, viral genomes are represented in a very low proportion of sequencing reads in a shotgun approach due to their small genomes compared to cellular genomes. Viral particles should be purified to obtain a complete analysis of the viral community (both DNA and RNA viruses), and this cannot be done from the vaseline strip without introducing some important biases (Prussin AJ., et al., 2014, FEMS Microbiol Lett, 357:1-9; Thurber RV., et al., 2009, Nat Protoc 4:470-483). Additionally, Hirst-type samplers are expensive devices and usually not portable, so its usage is limited to a restricted number of sampling sites simultaneously.

Polytetrafluorethylene (PTFE) filters have been also tested for collecting airborne bacteria, virus and other particles in the 10-900 nm size range (Nancy C. B., et al., 2007, Ann. Occup. Hyg., 51: 2, 143-151), however, no efficient analysing method has been proposed for identifying the whole captured biological particles.

Therefore, there is a need for developing improved capturing and analysing methods which use devices and methodologies that allow to collect and study the whole airborne biological community, including viruses and other important pathogens and allergens, in an efficient way.

### DESCRIPTION OF THE INVENTION

The present invention refers to a device for capturing air biological particles which comprises polytetrafluorethylene (PTFE) filters and its use for collecting, detecting and identifying organisms present in air ecosystems. The invention also provides a method suitable for the capture, detection and identification of whole airborne biological particles, including viruses and other important air pathogens and allergens, with the use of the device comprising the PTFE filters and preferably a sequencing shotgun metagenomic approach. This method allows to perform organism, preferably viral, metagenomics to sequence all DNA and RNA organisms captured in the filters, and/or to amplify specific genomes by gene amplification methods. This methodology may be applied to detect, for instance, SARS-CoV2 particles in air samples.

The study of the airborne biological community, composed by very different organisms (such as bacteria, fungi, pollen and virus), requires the use of an efficient sampling method that captures a good representation of the whole community. In the present invention, inventors have analysed the microbial community captured with seven different air samplers, based on different capturing methods (impact samplers, cyclonic, impinger and filters), to determine which is the most suitable to obtain a more complete view of the airborne biological community and to establish it as a standarized method.

Thus, in the present invention different methods for collecting airborne microorganisms, including impactor samplers, liquid impingers, cyclonic samplers and different filters, have been tested. Additionally, to avoid the biases of culture-dependent methods or amplicon-based analyses, the inventors have performed a next generation sequencing (NGS) shotgun approach to uncover the whole airborne diversity. A detailed analysis of the genetic diversity of the airbiota captured using these different methodologies is presented in the Examples shown below. Therefore, the Examples of this application provide a detailed comparison of the biodiversity detected by seven different air sampling devices, with the analysis of shotgun metagenomics and amplicon sequencing of prokaryotic/eukaryotic organisms to evaluate the biodiversity captured by the different samplers. The results obtained evidence that, while isolation and purification of virus particles is not possible from many devices, PTFE filters are the best option for capturing and analysing viral metagenomics and thus these filters are the methodology of choice for performing the most efficient sampling and analysing method that captures the best representation of the whole airborne organisms community.

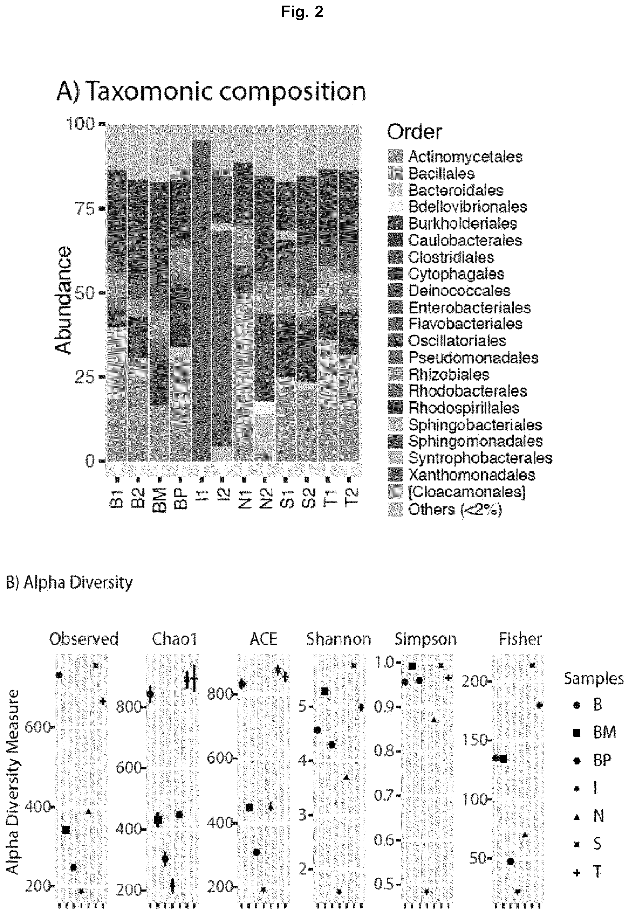

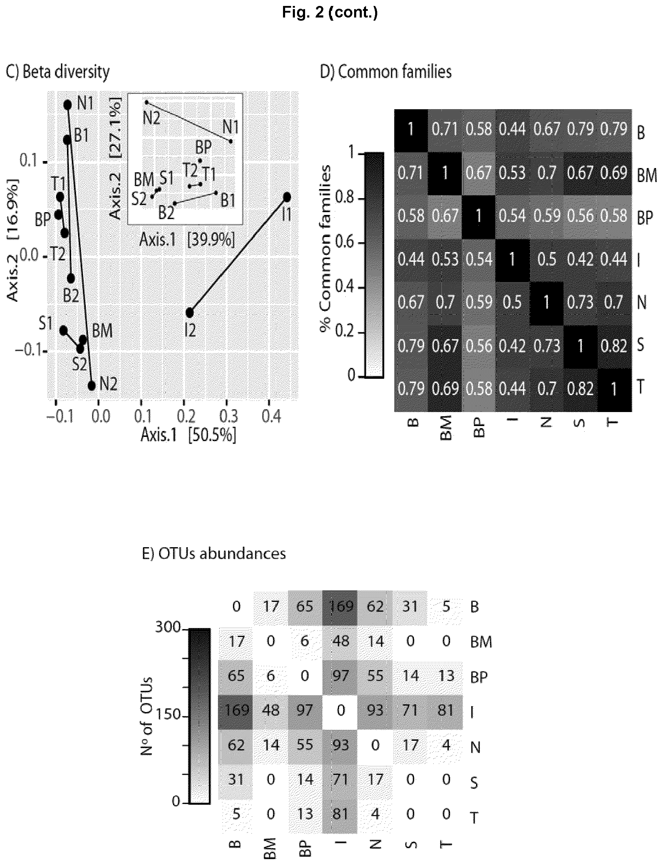

Consequently, the Examples shown below, which compare airbiota analysis from different air samplers, evidence that PTFE filters show the best efficiencies capturing microbial diversity from the air (Figures 2-4).

PTFE filters have also the following advantages: they are cheaper than others samplers, replicates can be sampled using two or more filter holders, and several localizations (both outdoor and indoor) can be studied simultaneously using several devices with a reduce cost. Additionally, taking into account other factors such as cost and portability, PTFE filters were the most convenient device of those tested. Finally, it has been demonstrated herein that PTFE filters allow the extraction of the viral fraction for viral metagenomics, which allows the analysis of the whole airborne biological community. Consequently, PTFE filters represent the best option for performing viral metagenomics because of the easy to extract cellular organism and virus particles by shaking or sonication of the filters in a buffer.

Therefore, in a first aspect, the present invention relates to a device for capturing air biological particles, hereinafter "the device of the invention", comprising:
- a substrate configured to capture particles from flowing air thereon using at least one captor;
- at least one processor operable to enable the air to flow through or over the substrate, thereby causing the particles in the air to be captured by the substrate;
wherein the substrate and the at least one processor are communicatively coupled.

Preferably, the substrate comprises at least one filter acting as the at least one captor.

Preferably, the at least one filter is a polytetrafluorethylene (PTFE) filter.

Preferably, the polytetrafluoroethylene (PTFE) filter is a synthetic fluoropolymer of tetrafluoroethylene filter.

In a preferred embodiment of the device, the PTFE filter has a nominal pore size between 0.2 µm and 5 µm, preferably 1 µm or 5 µm, more preferably of 1 µm. A PTFE filter with a nominal pore size of 1 µm is specially advantageous for collecting viral particles from air.

Optionally, the device for capturing air biological particles further comprises an air pump operable to increase or decrease flow of air,
wherein, to capture the particles into the substrate, the at least one processor is operable to:
cause the air pump to increase or decrease the flow of air containing the particles toward the substrate.

Preferably, the air pump is a vacuum pump.

Another aspect refers to the use of the device of the invention for collecting or capturing or sampling, and for detecting and identifying organisms present in air (including outdoor and indoor air), preferably wherein the detection and identification are performed by metagenomic analysis, more preferably by a sequencing shotgun metagenomic approach. Alternatively, by amplification of specific genomes with the use of specific primers and gene amplification methods, followed by sequencing.

The invention also refers to a system for detecting and identifying organisms present in the air comprising the above explained device for capturing air biological particles.

Preferably, the system for detecting and identifying organisms present in the air further comprises centrifugal means configured to remove cellular organisms from the substrate, and buffering means configured to house the substrate.

Preferably, the system for detecting and identifying organisms present in the air further comprises filtration means configured to filtrate the supernatants containing the virus particles, wherein the centrifugal means are also configured to concentrate the supernatants.

Preferably, the system for detecting and identifying organisms present in the air further comprises nuclease treatment means configured to remove all non-encapsidated DNA or RNA.

Preferably, the system for detecting and identifying organisms present in the air further comprises gene amplification and sequencing means for the amplification of collective or specific viral genomes captured in the filters, followed by sequencing.

In another more preferred embodiment, the detection and identification of organisms present in air, referred to in the present invention, is performed by metagenomic analysis, more preferably by a next generation sequencing (NGS) shotgun approach.

In another more preferred embodiment, for sequencing and detection of specific viruses, the viral genomes or a specific gene are amplified by PCR with specific primers and the product is identified by quantitative (qPCR) or quantitative reverse transcription PCR (RT-qPCR), or sequenced using Illumina HiSeq 200 machine or equivalent.

In another preferred embodiment the system for detecting and identifying organisms present in the air comprises the device of the invention and further comprises centrifugal means configured to remove cellular organisms from the substrate, buffering means configured to house the substrate filtration means configured to filtrate the supernatants containing the virus particles, wherein the centrifugal means are also configured to concentrate the supernatants, nuclease treatment means configured to remove all non encapsidated DNA or RNA and gene amplification and sequencing means for the amplification of collective or specific viral genomes captured in the filters, followed by sequencing.

The organisms present in air or in air samples will be also referred to in the present invention as "airborne organisms", "airbiota" or "airborne biological community". These organisms are, preferably, microorganisms, more preferably, the organisms are selected from the list consisting of: viruses, including DNA and RNA viruses, plants, bacteria, pollen and fungi. Even more preferably, the organisms are air pathogens, particularly viruses, preferably SARS-CoV2.

"Air pathogens" are those organisms present in the airbiota which are capable of trigger a disease, an infection, a clinical condition, and the like, in humans or non-human animals. Examples of "air pathogens" are, but without limitations, bacteria such as mycobacterium tuberculosis, bordetella pertussis and mycoplasma pneumpniae, and viruses such as influenza virus, coronavirus, adenovirus, varizela zoster virus and respiratory syncitial virus.

Thus, another aspect of the invention refers to the use of the device of the invention for collecting, detecting and identifying air organisms, hereinafter "the use of the invention".

In a preferred embodiment of the use of the invention the air organisms are selected from the list consisting of: viruses, plants, bacteria, pollen and fungi.

In another preferred embodiment of the use of the invention the organisms are air pathogens, preferably virus pathogens, more preferably the virus is SARS-CoV2.

Another aspect of the invention refers to a method, hereinafter "the method of the invention", for collecting, detecting and identifying organisms, preferably microorganisms, present in the air, or for the analysis of airbiota, wherein said method comprises the steps of:
a. sampling an air sample with the device of the invention,
b. extracting the genetic material comprised in the device after step (a),
c. randomly amplifying the genetic material extracted in step (b), and
d. sequencing, preferably by metagenomic analysis, more preferably by a sequencing shotgun metagenomic approach, the genetic material amplified in step (c).

The sampling of step (a) may be performed during at least 1 hour, preferably at least one day, more preferably during at least 4 consecutive days, even more preferably during at least 8 consecutive days, even more preferably during at least 12 hours per day.

The genetic material of step (b) may be DNA or RNA, single or double stranded, circular or lineal. This genetic material may be extracted by conventional DNA or RNA extraction methods well-known in the art.

Amplification of step (c) is preferably carried out by multiple displacement amplification (MDA), more preferably using primers for 16S rRNA, for bacteria, and Internal transcribed spacer 2 (ITS2), for plants and fungi.

In another preferred embodiment, the metagenomics analysis of step (d) is performed by an NGS shotgun approach, more preferably using an Illumina HiSeq 2000 machine or equivalent.

In another preferred embodiment, the method of the invention comprises an alternative step c (c') by amplification of specific genomes with the use of specific primers and gene amplification methods, followed by sequencing.

For sequencing and detection of specific viruses of step (c'), the viral genomes or a specific gene are amplified by PCR with specific primers and the product is identified by quantitative (qPCR) or quantitative reverse transcription PCR (RT-qPCR), or sequenced using Illumina HiSeq 200 machine or equivalent.

In another preferred embodiment of the method of the invention, the organisms are selected from the list consisting of: viruses, including DNA and RNA viruses, plants, bacteria, pollen and fungi. Even more preferably, the organisms are air pathogens, particularly viruses, preferably the SARS-CoV2 virus.

Thus, the method of the invention is useful for collecting, detecting and identifying air pathogens.

In another preferred embodiment, the method of the invention comprises an additional step (a'), between steps (a) and (b), in which cellular organisms are removed from the device, more preferably from the PTFE filter comprised in the device. Previously to this step (a'), the PTFE filter comprised in the device may be optionally vortex, more preferably at maximum speed for 1 h at 4°C.

The cellular organisms may be removed by, for instance but without limitation, resuspending the airborne particles captured in the filter in saline buffer, followed by centrifugation, shaking or sonication, and finally filtration. Afterwards, a concentration step of the supernatants containing the virus particles may be performed.

Furthermore, preferably, an additional step after step (a') and before step (b), is carried out, which comprises removing the non-encapsidated DNA or RNA, more preferably with a nuclease treatment.

Methods and uses described in the present invention may be carried out in air samples collected from indoor or outdoor.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

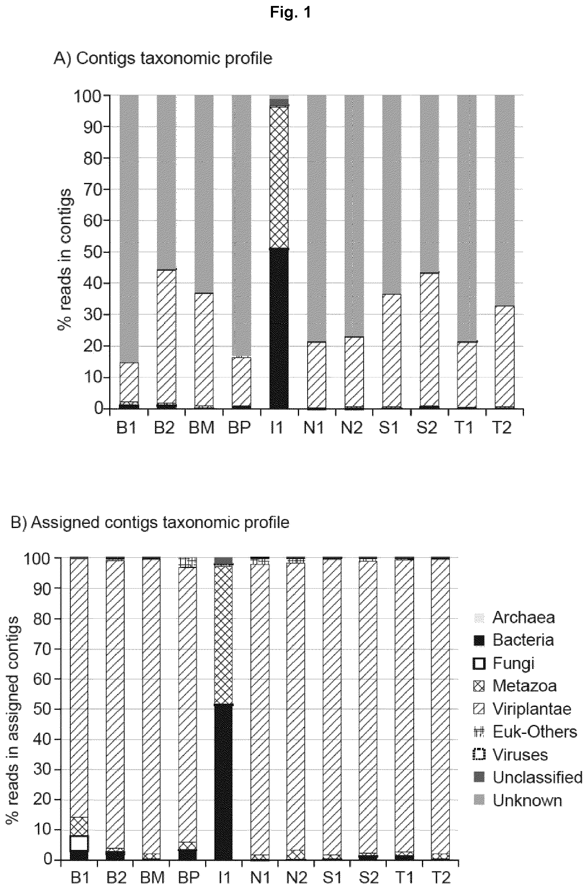

**Fig. 1****. Contigs taxonomic classification.** Contigs were first aligned using Blastn against NT database. Unassigned contigs were then aligned using Blastx against NR database. Quality filtered reads were mapped against all contigs using BWA. The percentage of mapped reads is represented, considering all contigs (A) or assigned contigs (B).
**Fig. 2****. Bacteria metataxonomic analysis.** A) Taxonomic profile at the order level. Orders with abundances lower than 2% were merged. B) Alpha diversity indexes plot. Replicates, when available, were merged for this analysis. Samples are indicated as follow: B, Burkard; DM, Burkard Multivial; BP, BioPump; I, impiger; N, Nanoparticles; S, SAS; T, PTFE. C) Beta diversity analysis. Normalized counts were used for a PCoA analysis based on weighted UNIFRAC distances. A second analysis was done removing Impinger samples (small square). D) Heatmap showing the percentage of common taxa (at family level) shared by the different samples. E) Number of OTUs with significant differences in their abundances (q-value < 0.1).

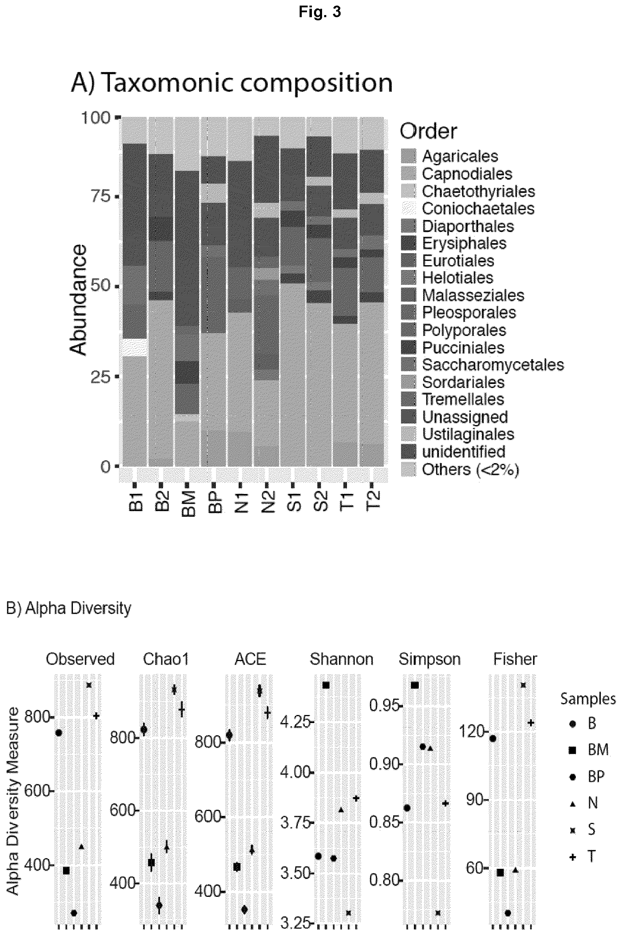

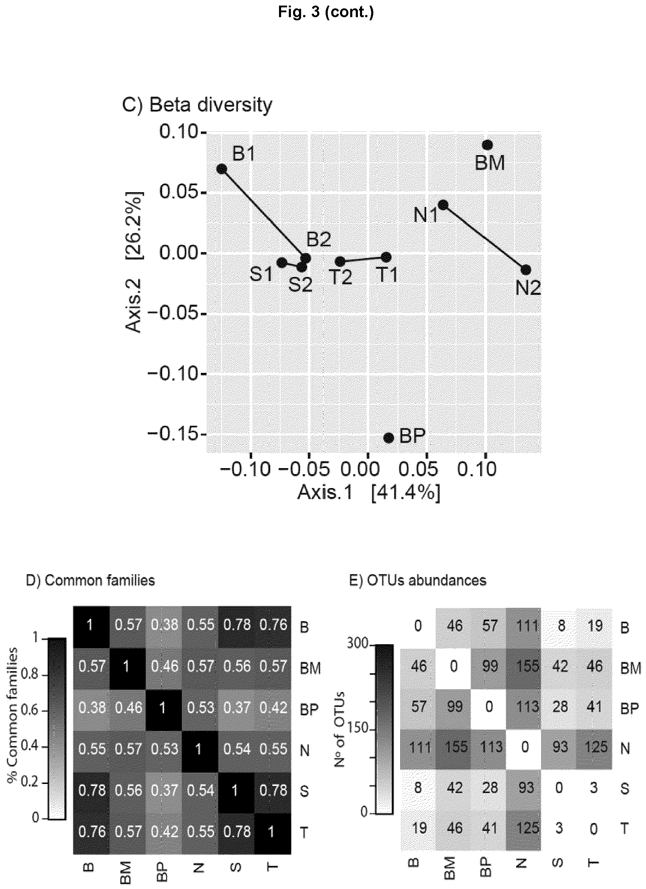

**Fig. 3****. Fungal metataxonomic analysis.** A) Taxonomic profile at the order level. Orders with abundances lower than 2% were merged. B) Alpha diversity indexes plot. Replicates, when available were merged for this analysis. Samples are indicated as follow: B, Burkard; DM, Burkard Multivial; BP, BioPump; I, impiger; N, Nanoparticles; S, SAS; T, PTFE. C) Beta diversity analysis. Normalized counts were used for a PCoA analysis based on weighted UNIFRAC distances. D) Heatmap showing the percentage of common taxa (at family level) shared by the different samples. E) Number of OTUs with significant differences in their abundances (q-value < 0.1).

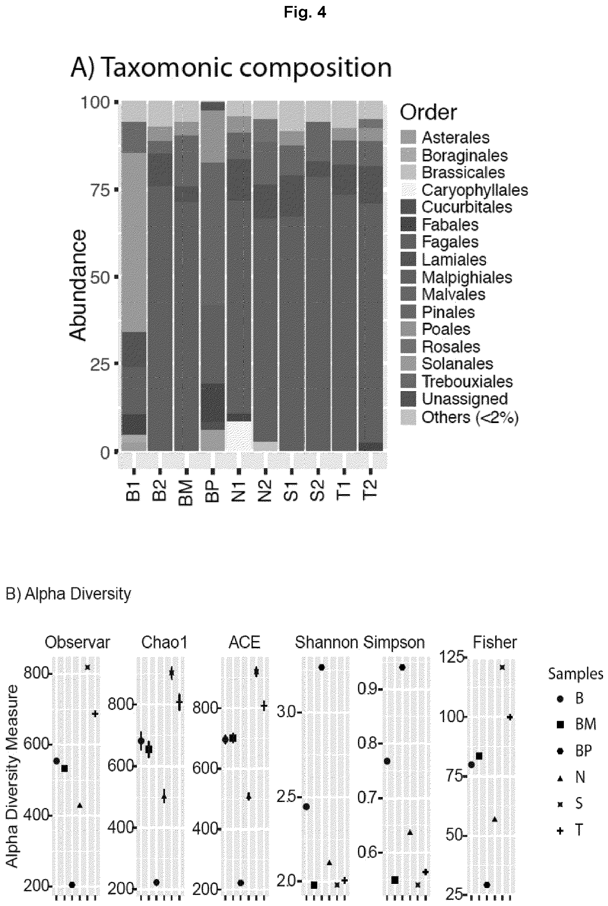

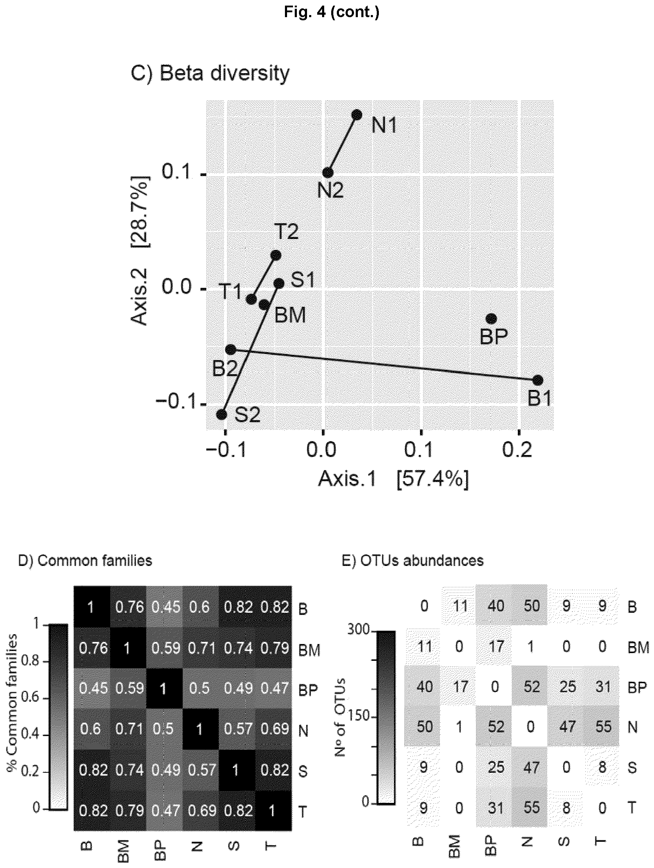

**Fig. 4****. Plants metataxonomic analysis.** A) Taxonomic profile at the order level. Orders with abundances lower than 2% were merged. B) Alpha diversity indexes plot. Replicates, when available were merged for this analysis. Samples are indicated as follow: B, Burkard; DM, Burkard Multivial; BP, BioPump; I, impiger; N, Nanoparticles; S, SAS; T, PTFE. C) Beta diversity analysis. Normalized counts were used for a PCoA analysis based on weighted UNIFRAC distances. D) Heatmap showing the percentage of common taxa (at family level) shared by the different samples. E) Number of OTUs with significant differences in their abundances (q-value < 0.1).

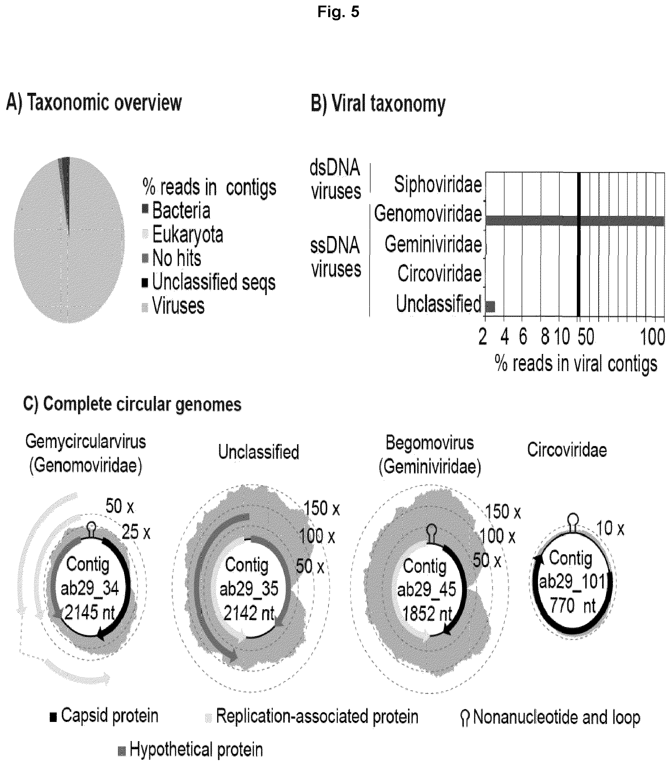

**Fig. 5****. PTFE virome analysis.** A) General overview of taxonomy. Contigs were aligned against NCBI NR database using BlastX (the percentage of reads in contigs is represented). B) Viral contigs classification (BlastX against NR viral protein database). C) Complete circular viral genomes detected. Coverage is represented as a grey histogram around genome scheme.

### EXAMPLES

### 1. Material and methods.

### Sampling

Several sampling devices based on different capturing mechanisms were chosen to compare airborne biological particles (ABPs) capture: three impact/slit samplers (Hirst spore trap sampler (Burkard), Surface Air System DUO 360 (SAS), and Zefon Bio-Pump^{®} Plus (Biopump)), a cyclonic device (Burkard Multivial), a custom impinger and two different filters (Polytetrafluoroethylene (PTFE) and Glass fiber (GFC, 1.7 µm) sandwich with and inner layer of nanoparticles). Hirst spore trap sampler (Burckard) is an impact sampler based on the capture of vacuum-accelerated airborne particles in a vaseline-covered strip. Surface Air System DUO 360 (SAS) was designed for the direct culture of the microorganisms captured from air as is equipped with two heads where agar plates are placed. To reduce the culture bias we replaced the agar plates by vaseline-covered Petri dishes (new plates were used for each sampling day). Zefon Bio-Pump ^{®} Plus (Biopump) uses cartridges containing a slide covered by a patented aqueous solution to capture the ABPs which is easier to recover after the sampling (1 cartridge was used per sampling day). Burckard Multivial uses vacuum suction to create a cyclon inside a 1.5 ml tube where ABPs are deposited due to the centrifugal force. A new sterilized 1.5 ml tube was used every day. The custom impinger was built with gas washer bottles (100-200 µm nominal pore size) to capture ABPs by the interchange of particles between the air a buffered solution (MSM buffer: 50 mM Tris-HCI, 100 mM NaCl and 8 mM MgCl₂, pH 7.5). For each sampling day a 500 ml bottle of autoclaved buffer were used. Finally, two different filters were used: PTFE (5 µm nominal pore size) and a GFC (1.7 µm, Whatman) sandwich with an inner layer of iron-coated particles (MIL-100(Fe) nanoparticles with 1-10 µm pore size). A GFC-nanoparticle sandwich was prepared directly onto the filter holders. First, a GFC filter was added before injecting an ethanol solution containing a suspension of nanoparticles (-40 mg). Then, a second GFC filter was added and the sandwiches were dried at 100°C for 24 h. Filters were reused for the four samplings days. We used duplicates when enough devices were available (Table 1). Sampling was carried out in the roof of the Escuela Técnica Superior de Ingenieros Industriales, Universidad Politécnica de Madrid (Spain, 40.439881°N, 3.689409°W, ca 30 m over the street). All samplers were run according to device characteristics (Table 1). Air was sampled during 12 h per day during four consecutive days in May of 2016. Biopump was only run during 10 h due to software limitations, and SAS was only run during 20 min, 3 times per day, to reduce the total volume capture for comparison purpose, since its flow rate is very high (150 L/min) with respect to the other samplers.

**Table 1. Samplers summary.**

| **ID** | **Sampler** | **Flow (I x min⁻¹)** | **Time¹** | **Total air volume (m³)** |
|---|---|---|---|---|
| B1 | Burckard | 10 | 12 hours/day | 28.8 |
| B2 | Burckard | 10 | 12 hours/day | 28.8 |
| BM | Burckard Multivial | 15 | 12 hours/day | 43.2 |
| BP | Biopumb | 15 | 10 hours/day | 39 |
| I1 | Impinger | 6-6.5 | 12 hours/day | 17.3 |
| I2 | Impinger | 4.5-5 | 12 hours/day | 13 |
| N1 | Nanoparticles | 5.5 | 12 hours/day | 37.3 |
| N2 | Nanoparticles | 11 | 12 hours/day | 15.8 |
| S1 | SAS | 150 | 1 h/day, 3x20 min (9,13,17h) | 43.2 |
| S2 | SAS | 150 | 1 h/day, 3x20 min (9,13,17h) | 43.2 |
| T1 | PTFE filter | 11-12 | 12 hours/day | 33 |
| T2 | PTFE filter | 11-13 | 12 hours/day | 34.5 |

| | | | | |
|---|---|---|---|---|
| ¹ Sampling was repeated during 4 consecutive days. | | | | |

### DNA extraction

After sampling, total DNA was directly extracted from all devices using PowerMax^{®} Soil DNA Isolation Kit (MO BIO Laboratories), except in the case of impingers that were subjected to tangential flow filtration (70 kDa cartridge) to reduce sample volume from 2 L to -10 mL. Vaseline from Burkard and SAS sampler were recovered using a sterile blade and subjected to direct DNA extraction. The aqueous solution of the four Biopump cartridges used, one per day, were recovered and mixed for DNA extraction. ABPs captured by the Burkard Multivial were resuspended in MSM buffer before DNA extraction. Filters (PTFE and GFC) were added directly to DNA extraction tubes. The eluted DNA (5 mL) was then ethanol precipitated. Briefly, 200 µL of 5 M NaCl, 2.5 µL of linear polyacrylamide as carrier (LPA, 25 µg/µL, Sigma) and 10.5 mL of cold absolute ethanol were added. Samples were centrifuged for 30 min at 2500 x g. Pellets were washed with cold 70% ethanol and air dried. DNA was resuspended in 100 µl of nuclease free water (Ambion). All procedures were carried out in a UV-cabinet (BioSan UVT-B-AR) and all equipment was treated with 0.1 M HCI solution to avoid contaminations. Two mock DNA extractions were included to test for putative contaminations during extraction. Samples were also tested for the presence of microorganism by PCR using different marked genes (16S rDNA and ITS2) (Table 2). No amplification was observed in mock samples.

**Table 2. Primers used for amplicon sequencing and contamination controls.**

| **Primer** | **Sequence (5->3)** | **Target** | **Reference** |
|---|---|---|---|
| BacF | CCTACGGGNGGCWGCAG (SEQ ID NO: 1) | 16S rDNA | Takahashi S, Tomita J, Nishioka K, Hisada T, and Nishijima M. |
| BacR | GACTACHVGGGTATCTAATCC (SEQ ID NO: 2) | | 2014. PloS One 9 (8):e105592 |
| ITS86F | GTGAATCATCGAATCTTTGAA (SEQ ID NO: 3) | Fungal ITS2 | Op De Beeck M, Lievens B, Busschaert P, Declerck S, Vangronsveld J, and Colpaert JV. 2014. PloS One 9 (6):e97629. |
| ITS4 | TCCTCCGCTTATTGATATGC (SEQ ID NO: 4) | | |
| ITSD-For | YGACTCTCGGCAACGGATA (SEQ ID NO: 5) | Plantae ITS2 | White, TJ, Bruns TD, Lee SB, Taylor JW, and John S. 1990. In PCR Protocols, 31:315-22. Academic Press, Inc. |
| ITS4-Rev | TCCTCCGCTTATTGATATGC (SEQ ID NO: 6) | | |

### Shotgun sequencing and analysis

Total DNA samples were randomly amplified using multiple displacement amplification (MDA, GenomiPhi kit, GE Healthcare). 10 µL of each sample were used for amplification following manufacturer's instructions. Samples were amplified for 2.5 h, except for B1 and I1 that required 3.5 h of amplification to obtain sufficient DNA for sequencing. Sample I2 was discarded because no amplification was observed after 6 h. No amplification was observed in mock samples. Library preparations and sequencing were performed at Centro Nacional de Análisis Genómico (CNAG, Barcelona, Spain). Sequencing was done using an Illumina HiSeq 2000 machine obtaining -40 M paired-end reads (2x126 pb) for each sample. Raw reads were quality filtered using PRINSEQ (minimum read length 100 pb and minimum average quality 25). Orphan reads were discarded. Taxonomy binning of reads was carried out using Centrifuge against NCBI non-redundant nucleotide database. Only reads uniquely assigned with a score higher than 200 were considered. Reads assigned to Human or PhiX174 were excluded. Centrifugeassigned reads were normalized using metagenomeSeq and used for beta diversity analysis using PhyloSeq. Quality filtered reads were then assembled with IDBA_UD (--pre_correction --mink 20 --maxk 120 --min_contig 500). Assembled contig with low complexity were removed using PRINSEQ (-lc_method entropy -lc_threshold 70). A hybrid-Blast approach was used for contig taxonomic classification. First, contigs were aligned using Blastn against NCBI non-redundant nucleotide database (NT, downloaded on May 2017). Only those hits with e-value < 1e-3 and score > 50 were considered, and best hit was assigned. Contigs with no hits or with hits with e-value > 1e-3 and score < 50 were then subjected to Blastx alignment against NCBI non-redundant protein database (NR, downloaded on May 2017). Again, best hit was used for taxonomic assignments (e-value <1e-3 and score < 50). Reads were mapped to contigs using BWA MEM.

### Metataxonomic sequencing and analysis

In order to obtain a taxonomic profile of the different sampling devices we used a targeted metagenomic approach. MDA-unamplified DNA samples were use as template for marker genes amplification. We used 16S rRNA for bacteria and Internal transcribed spacer 2 (ITS2) for plants and fungi (Table 2 above). PCR amplification and library preparation were carried out at Parque Científico de Madrid (Madrid, Spain). Briefly, 100 pg of DNA, quantified using Picogreen, were used for a first PCR using marker genes primers linked with Illumina adaptors (CS1 and CS2). The PCR conditions were as follow: 98°C for 30 min, followed by 26 cycles of denaturation at 98°C for 30 s, 50°C for 20 s, 72°C for 20 s, followed by a final extension at 72°C for 2 min. A second PCR of 8 cycles were performed using a 1/25-1/200 dilution of the first one using the same cycling conditions but using CS1 and CS2 primers linked to different barcodes and additional Illumina adapters also required for sequencing (p5 and p7). Q5^{®} High-Fidelity DNA Polymerase (New England Biolabs) were used for PCRs. Amplified products were quantified and pooled before sequencing in a MiSeq machine obtaining -200,000 reads (2x300pb). Raw reads were quality filtered as described above and paired reads were joined and adapters were removed using PANDAseq. Taxonomic assignments were performed using Qiime software. The Greengenes database (version gg_13_8 implemented in Qiime) was used for bacterias and UNITE (version no. 7.1) was used as fungal database. A custom database was used for plant classification. OTUs were defined at 97% sequence similarity and only those with at least 5 counts and present in at least 2 samples were kept for further analysis. Chloroplast and mitochondrion OTUs were removed from bacterial analysis. Although specific ITS2 primers for plants or fungi were used, some cross-amplification can be produced, and therefore fungal OTUs were removed from plant analysis and plant OTUs from fungal analysis. OTU counts were normalized using the metagenomeSeq method. Taxonomic profile, diversity indexes and beta diversity analysis were performed on normalized counts using Phyloseq package. Common family analysis were performed after merging replicates using Phyloseq. A dedicated Qiime script (differential_abundance.py) was employed to analysis the significance of OTU abundance differences using DESeq2 method.

### Virus sampling, sequencing and analysis

For virus analysis we used PTFE filters (1 µm, PALL Zefluor). A vacuum pump equipped with 2 filter holders was placed on Alcobendas city (at 14 km from Escuela Técnica Superior de Ingenieros Industriales, Universidad Politécnica de Madrid) at 1.5 m over the ground. Filters were sampling for 8 days (from the 9^{th} to the 17^{th} of February of 2017) at 2.5 L/min. After sampling, filters were vortexed at maximum speed in 10 mL of MSM buffer for 1 h at 4°C. Cellular organisms were removed by centrifugation (20' at 3000xg) followed by 0.45 µm filtration of the supernatant. The supernatants containing the virus particles were then concentrated using centrifugal units (100 kDa, Amicon). Concentrated virus particles were subjected to nuclease treatment to remove all non-encapsidated DNA or RNA (200 U of DNAse I, 120 U of Nuclease S7 and 10 µg of RNAse A) for 1 h at RT. Viral DNA was extracted with the MinElute kit (QIAgen), following manufacturer's instructions. Samples were tested for the presence of cellular microorganism by PCR using different marked genes (16S rDNA and ITS2) (Table 2 above) and no amplification was observed neither in the samples nor in the mock sample (10 mL of MSM buffer that were subjected to all steps). Then, 10 µL of each sample were used to randomly amplify all DNA using GenomiPhi kit (GE Healthcare) for 2.5 h. We obtained amplification in both replicates, but not in mock sample. One of the replicates were sequenced at Parque Científico de Madrid using a MiSeq machine, obtaining 682,832 paired reads (2x300 pb). Raw reads were quality filtered using PRINSEQ (minimum read length 150 pb and minimum average quality 25). Paired quality filtered reads were used for assembly using SPAdes v3.9.0. Contigs shorter than 500 pb were discarded. Contigs were aligned using BlastX against the NCBI non-redundant protein database (NR). To remove any putative non-viral sequence, only the contigs with at least 20% of viral hits (e value <1e-3) were considered as viral. Contigs without any hit were also considered as putative viral sequences. Viral contigs were then classified using BlastX against a non-redundant viral protein database (extracted from the NCBI NR database using taxonomic id 10239). Best hits were assigned (e value < 1e-3 and score > 50). Sequencing reads were mapped against contigs using BWA MEM. Complete circular viral genomes were detected using Minimus2.

### Detection of SARS-CoV2 using PTFE filters

Five vacuum pumps (KNF) (D1-D5) equipped with 2 filter holder each were installed in La Paz hospital (Madrid, Spain). Two consecutive rounds of sampling were done; from 20/03/2020 to 23/03/2020 (Experiment 1, 3 days) and from 23/03/2020 to 27/03/2020 (Experiment 2, 4 days) (Table 5). Polytetrafluoroethylene (PTFE) filters were used for air sampling at a flow rate of 2.5 l/min. After sampling, filters were immediately immersed in 4 ml of TRIzol (Invitrogen). Three RNA extraction from 1 ml from TRIzol were performed following manufacturer's instructions. For each sample, 3 RNA pellet were resuspended together in 25 µl of Nuclease-Free water (Ambion). For all procedures, DNA Lobind tubes were used (Eppendorf).

SARS-CoV2 E gene was measure using a Taqman assay (Bio-Rad) containing primers and probe designed by Charité Hospital (Berlin) and approved by World Health Organization for clinical diagnosis (Table 3). A 2-step approach was applied. Briefly, 5 µl of RNA was retrotranscribed using the SuperScript IV First-Strand Synthesis System (Invitrogen). Then, 1 µl of cDNA was used as template in a 10 µl qPCR reaction using Taqman Fast Universal PCR Master Mix (Applied Biosystems). Thermal conditions were as follow: 20 seg at 95°C, 45 cycles of 5 seg at 95°C and 20 seg at 60°C. qPCR was performed in a ABI PRISM 7900HT SDS device (Applied Biosystems). Samples were evaluated using technical triplicates.

**Table 3: Primers used for SARS-CoV2 detection. FAM: 6-carboxyfluorescein; BBQ: blackberry quencher**

| **Primer** | **Sequence (5'>3')** |
|---|---|
| E_Sarbeco_F1 | ACAGGTACGTTAATAGTTAATAGCGT (SEQ ID NO: 7) |
| E_Sarbeco_R2 | ATATTGCAGCAGTACGCACACA (SEQ ID NO: 8) |
| E_Sarbeco_P1 | FAM-ACACTAGCCATCCTTACTGCGCTTCG-BBQ (FAM-SEQ ID NO: 9-BBQ) |

ORF1ab and N genes were measure in a one-step approach using the Novel Coronavirus (2019-nCoV) Nucleic Acid Diagnostic Kit (Sansure). Briefly, 2.5 µl and 5 µl of RNA from Experiments 1 and 2 respectively, were used in a 25 µl qPCR reaction. Thermal conditions were as follow: 30 min at 50°C for retrotranscription, 1 minute at 95°C followed by 45 cycles of 15 seg at 95°C and 30 seg at 60°C. One step qPCR was performed in a CFX-384 device (Bio-Rad).

### 2. Results.

### Shotgun sequencing approach

To obtain a global view of the airborne biological community without the restriction of targeted metagenomic approaches, that do not include viruses, we decided to sequence total DNA using a shotgun sequencing approach. However, the amount of total DNA recovered from the air samples was <1 ng/µl, and therefore we had to randomly amplify the DNA samples using Multiple Displacement Amplification (MDA) to obtain sufficient DNA for library preparation. All samples were amplified for 2.5 h, except by Burkard 1 (B1) and Impinger 1 (11) that required 3.5 h of amplification to obtain sufficient DNA. The Impinger 2 (12) sample was discarded because no DNA amplification was observed even after 6 h of MDA. Total DNA was directly sequenced using the Illumina technology, obtaining -40 million paired reads for each sample. Raw reads were subjected to quality filtering prior to contig assembly. We obtained on average 13,000 contigs per sample, being the impinger (11) the sample with fewer contigs, closely followed by the Biopump (BP) and one of the Burkard replicates (B1). By contrast, SAS, Burkard Multival (BM) and the other Burkard replicate (B2) showed the highest number of contigs. Total number of assembled bases was -18 million on average, but one of the Burkard samples (B1), the Biopump (BP), one of the GFC filters (N1) and the impinger (11) were clearly under this average which correlated with a reduced number of contigs. However, average contig length and N50 were similar among all samples except for I1 that was almost twice with respect to all other samplers.

Contigs were classified using both Blastn and Blastx against the NCBI non-redundant databases (Figure 1). On average 60% of the contigs were assigned successfully, that represents -70% of total assembled bases and -33% of the reads (Figure 1A). Nevertheless, most of the assigned contigs were classified as belonging to *Viridiplantae* (Figure 1B). This was an unexpected result taken into account previous reports where plant sequencing reads were less abundant than for instance bacterial sequencing reads, including samples collected in spring when the highest pollen concentration is found, suggesting a higher bacterial abundance. However, in our experimental set, plant sequencing reads represented on average -86% of total mapped reads in the assigned contigs. Excluding the I1 sample, the average percentage of plant sequencing reads increased to up to 94%, reaching up to 97% in several samples (BM, S1, T2). Furthermore, most contigs assigned as *Viridiplantae* were classified as *Pinales* (-80% on average). *Pinus* genome is one of the highest plant genomes sequenced to date (-22 Gb) and this could be the main reason of these unexpected results. One single copy of a *Pinus* genome would account for up to 99% of the reads if it was sequenced together with a single bacteria (4 Mb genome). Therefore, it seems that *Pinus* pollen have monopolized most of the sequencing reads. In order to verify this, quality filtered reads were directly classified using Centrifuge. Although sequencing reads contain less information than contigs, and therefore a small fraction of sequencing reads can be classified, the results showed a similar pattern. Most of the reads were assigned to *Viridiplantae* (-75% of assigned reads) and most of them belong to *Pinus* genus (-67%). Then, we used the assigned sequencing reads to compare different sampler through beta diversity analysis but the results are clearly dominated by plant reads. Sequencing reads assigned to different groups (Bacteria, Fungi and Viridiplantae) were analyzed independently, and the distribution observed using all reads was almost identical to the one observed using only plant reads. Bacteria plot, although inverted, was also similar, which could indicate an influence of this group in the ordination of the samples. By contrast, fungal sequencing reads analysis was more dispersed, being grouped by replicates, which could indicate an influence in the capture of fungal spores depending on the sampler. However, the number of non-plant reads was very small for a shotgun analysis to be properly compared, and therefore we must be cautious. Nevertheless, we could observe a clear contamination in sample I1 (Figure 1) that, in contrast to other samples, was dominated by bacterial and metazoan reads, being the assigned reads almost 100%. Most of the bacterial assigned reads were related to *Alphaproteobacteria* (*Rhodobacteraceae*) that most likely grew in the impingers during the 12 h of sampling.

Although the results of these shotgun experiments are dominated by plant sequencing reads, mainly from *Pinus* genus, we were able to detect some viral contigs (Table 4).

**Table 4. Taxonomic profile of the assigned viral reads. Numbers represent the number of mapped reads in viral contigs.**

| **Genome** | **Order** | **Family** | **B1** | **B2** | **BM** | **BP** | **I1** | **N1** | **N2** | **S1** | **S2** | **T1** | **T2** |
|---|---|---|---|---|---|---|---|---|---|---|---|---|---|
| dsDNA viruses | *Caudo virales* | *Myoviridae* | 0 | 0 | 0 | 0 | 6275 | 0 | 0 | 0 | 0 | 0 | 0 |
| | | *Podoviridae* | 0 | 0 | 0 | 0 | 54 | 0 | 0 | 0 | 0 | 0 | 0 |
| | | *Siphoviridae* | 562 | 0 | 0 | 0 | 3963 | 0 | 0 | 0 | 41 | 0 | 0 |
| | - | *Baculoviridae* | 0 | 39 | 0 | 0 | 241 | 63 | 85 | 1409 | 4335 | 604 | 295 |
| | - | *Mimiviridae* | 0 | 0 | 0 | 0 | 0 | 0 | 0 | 69 | 0 | 1865 | 0 |
| | - | *Nudiviridae* | 0 | 0 | 0 | 0 | 0 | 0 | 0 | 0 | 0 | 303 | 0 |
| | - | *Phycodn aviridae* | 0 | 0 | 0 | 0 | 0 | 0 | 0 | 0 | 74 | 0 | 0 |
| | - | *Others* | 81 | 0 | 0 | 0 | 418 | 0 | 0 | 0 | 0 | 0 | 0 |
| Retro-transcribing viruses | - | *Caulimoviridae* | 0 | 4946 | 2983 | 0 | 0 | 0 | 17 | 3582 | 3015 | 169 | 708 |
| | - | *Retroviridae* | 0 | 0 | 0 | 0 | 0 | 0 | 33 | 0 | 0 | 0 | 0 |
| ssDNA viruses | - | *Circoviridae* | 89 | 0 | 0 | 0 | 0 | 0 | 0 | 0 | 0 | 0 | 0 |
| | - | *Genomo viridae* | 0 | 0 | 0 | 0 | 0 | 0 | 0 | 544 | 0 | 0 | 0 |
| | - | *Nanoviridae* | 0 | 0 | 0 | 0 | 0 | 0 | 0 | 28 | 0 | 0 | 0 |
| | - | *Others* | 0 | 0 | 0 | 0 | 161 | 0 | 0 | 0 | 0 | 0 | 0 |
| environmental samples | - | - | 0 | 0 | 0 | 0 | 461 | 0 | 0 | 0 | 0 | 0 | 0 |
| ssRNA viruses | - | *Flaviviridae* | 0 | 0 | 0 | 0 | 0 | 0 | 204 | 0 | 0 | 0 | 0 |
| unclassified bacterial viruses | - | - | 0 | 0 | 0 | 0 | 17102 | 0 | 0 | 0 | 299 | 0 | 0 |
| unclassified viruses | - | - | 0 | 0 | 281 | 0 | 0 | 0 | 23 | 0 | 0 | 0 | 0 |
| Total reads in contigs | | | 732 | 4985 | 3264 | 0 | 28675 | 63 | 362 | 5632 | 7764 | 2941 | 100 3 |

Most of them were classified as *Baculoviridae* and *Caulimoviridae,* insect and plant viruses respectively. We were also able to detect some bacteriophage (*Caudovirales*) contigs in sample I1, which correlates with a bacterial contamination in that sample, although some of them could represent prophages rather than free virus. Among viral contigs we were able to detect 5 complete circular viral genomes: one *Circoviridae-like* contig detected in B1, one *Genomoviridae-like* virus detected in S1 and three *Caulimoviridae-like* virus detected in B2, BM and S1. Another near-complete *Caulimoviridae-like* virus was also detected in S2 sample, and many caulimo-related contigs were also detected in other samples. Moreover, using the *Caulimoviridae*-like virus detected in B2 (contig B2_191) as reference we were able to detect reads from this virus in almost all samples except in I1. Samples B1 (Burkard) and BP (BioPump) showed very few *Caulimoviridae-like* reads compared to the other samples. These *Caulimoviridae-like* viruses have been demonstrated to infect *Pinus* and could represent the first gymnosperm-infecting *Caulimovirus* described to date.

### Metataxonomic approach (targeted metagenomics)

Due to the *Viridiplantae* dominance in shotgun sequencing and the impossibility of doing a convenient comparative analysis, we decided to use the unamplified DNA samples to amplify marker genes to obtain a taxonomic profile of the microorganisms captured by the different sampling devices. We used 16S rRNA for bacteria and ITS2 for plants and fungi. Amplicons were sequenced in a MiSeq device obtaining -200,000 paired sequencing reads (2x300 pb) from each sample. Using this approach, we were able to amplify 16 rRNA from both impinger samples, however we did not obtain amplification of ITS2 (either from fungi or plants) from these two samples neither using a higher number of PCR cycles (data not shown). This result was consistent with the bacterial contamination observed in the shotgun analysis of these samples.

### Bacteria analysis

As mentioned above, Impingers taxonomic profile were mostly composed by *Rhodobacterales* from *Paracoccus* genus arising up to -95% of the reads in sample I1 (Figure 2A). This genus is also present in all other samples but with an average abundance of only 3%, and therefore the increased presence on Impingers is likely to be a contamination caused by the favourable growing conditions that this kind of samplers have (a liquid buffered solution). All other samples showed a similar taxonomic profile among them (Figure 2A) with exception of the Nanoparticle filters which were the more dissimilar between replicates (N1 and N2) and among samples. The huge differences observed between N1 and N2 could be due to the air flows that were substantially different between them (Table 1 above). Both filters were attached to the same vacuum pump and due to differences in filter setting air retention was stronger on one of them (N1) and most of the vacuum pressure went to the other filter (N2). *Bacillales,* mostly composed by soil-dwelling *Alicyclobacillus* genus, was a relative abundant group observed in most of the samples (excluding Impingers). However, no or very few bacteria from this order/genus were observed in Burkard Multivial and in SAS replicates, and Burkard samples showed a great discrepancy in its relative abundance (B1 -21.3% versus B2 -5.6% at order level).The differences observed between Burkard replicates could be caused by air flow variations during sampling such as in Nanoparticles, as the air flow was only tested at the beginning and at the end of every sampling day (not observing significant differences). By contrast, PTFE filters showed a very similar profile between replicates in spite of a small air flow differences (Table 1).

Regarding the captured diversity, Burkard (B), SAS (S) and PTFE (T) showed the highest value in almost all indexes (Figure 2B). By contrast, impingers (I) were the worst as only a very limited number of OTUs could be detected due to the contamination. Burkard Multivial (BM), Nanoparticles (N) and BioPump (BP) showed a reduced diversity. However, in beta diversity analysis all samples clustered together except the impingers (Figure 2C). When impingers were removed from the analysis, Nanoparticles appeared far from all other samples (Figure 2C inset), as this samples showed the most different taxonomic profile. The rest of samples were clustered together being SAS and PTFE replicates the most closely ordinated between replicates, while Burkard were more dispersed. Similarly, when common families were analysed, SAS, PTFE and Burkard showed the highest percentages, followed by Nanoparticles, Burkard Multivial and Biopump (Figure 2D). Again, Impingers only shared less than half of the detected bacterial families with the other samples. This data correlated well with the number of OTUs showing significant differences (Figure 2E). SAS, PTFE and BM did not show any significant difference. Burkard and Nanoparticles showed some differences with SAS, PTFE and BM, and more between them. Finally, Impinger showed the highest number of OTUs with significant differences with all other samples.

### Fungi analysis

The ITS2 region was amplified and sequenced for fungi taxonomic analysis. A general dominance of the order *Capnodiales,* most of them from *Cladosporium* genus, was observed in all samples (Figure 3A). Impingers were not included in the analysis because no amplification of ITS2 was observed even after increasing the number of PCR cycles, likely caused by the bacterial contamination. All other samples showed a similar taxonomic profile, and replicates were particularly good for SAS and PTFE. Some differences in the most abundant taxa could be observed. For instance, BM showed a highly reduced relative abundance of *Capnodiales,* and therefore a greater abundance of all others groups. This fact produced an increase in some of the diversity indexes (Shannon and Simpson) of this sample (Figure 3B). These index calculations rely on relative abundances (frequencies) that are more evenly distributed when an abundant taxa is not present or its relative abundance is substantially decreased. The second most abundant order, *Pleosporales,* where the relevant *Alternaria* genus belongs, showed a similar profile among all samples. By contrast, *Agaricales,* that were present in most samples, were almost absent in SAS replicates, in BM and in one the Burkard replicates (B1). B1, as seen in the bacterial analysis, showed a clear different profile to the B2 replicate (and with the rest of samplers), being absent some abundant taxas such *as Pucciniales or Erysiphales.*

However, Burkard, together with SAS and PTFE, showed high values in most diversity indexes (Figure 3B). The results of SAS in Shannon and Fisher indexes could be explained by the increased relative abundance of *Capnodiales,* that, unlike in BM, showed a reduction in these indexes. Apart from these differences in the taxonomic profiles, SAS, PTFE and Burkard (mainly B2) were clustered together when beta diversity was analysed. Nanoparticles, Burkard Multivial and BioPump were more dispersed probably due to differences in some taxa not captured by these samplers (such as *Erysiphales or Pucciniales*) (Figure 3C). The percentage of shared families and the number of OTUs with significant differences were consistently with beta diversity analysis (Figure 3D,E). SAS, PTFE and Burkard samples shared the highest number of families while SAS and PTFE showed a reduced number of OTUs with significant differences, being only 3 between SAS and PTFE (Figure 3E). BM, BP and N shared only -50% of the detected families with all other samples and showed a high number of OTUs with significant differences, being nanoparticles the most different (Figure 3E).

### Plants analysis

Plants were mainly capture in the samplers through pollen grain or small fragments present in the air. In general, the results of the analysis from plants showed a similar behavior than in bacterial and fungal analysis (Figure 4). Again, impingers were not included because no ITS2 amplification was observed. Most other samplers showed a dominance of *Fagales* (mainly *Quercus*) followed by *Lamiales* (*Plantago*) and *Pinales* (*Pinus*), in consonance with the pollinic profile of May in the city of Madrid. However, one of the Burkard replicates (B1) showed, such as in bacterial and fungal analysis, a clearly different profile. A single OTU assigned as *Solanum* (*Solanales*) account for more than 50% of assigned reads in B1 sample (19.178 reads) (Figure 4A). Nevertheless, this huge difference could be partially solved with the use of replicates, reducing the impact of this problem in the rest of the analysis (Figure 4). BioPump, that had no replicates due to resources availability, also had a discordant taxonomic profile, showed a divergent behavior in all analysis. It showed maximum diversity values in Shannon and Simpson parameters (Figure 4B) likely produced by a biased capture of pollen from *Fagales* and an increase on *Pinales,* that could influence the relative abundance of all other taxa, having a more even distribution increasing those diversity indexes. This phenomenon also affected partially to Burkard replicates, since the replicates average abundances are also influenced by the dominance of *Solanum* genus in B1. SAS and PTFE obtained high values in most of the diversity indexes, followed by Burkard and Burkard Multivial (Figure 4B). Consequently, these samples (SAS, PTFE, Burkard (B2) and Burkard Multivial) clustered together in the beta diversity plot (Figure 4C). BioPump, Burkard (B1) and Nanoparticles (both replicates) seemed to be more dispersed. SAS, PTFE and Burkard shared 82% of OTUs at family level and only 8 or 9 OTUs showed significant differences in their abundances among them (Figure 4D,E). Burkard Multivial (BM) showed a similar pattern to these three samplers, clustering together with them, although it showed a smaller diversity compared to them and a reduced number of shared families. However, this sampler did not show any OTUs with significant difference compared with SAS or PTFE and only 11 OTUs compared with Burkard (Figure 4E).

### Virome analysis

PTFE and SAS showed the best efficiencies capturing microbial diversity from the air (Figures 2-4). Burkard, our reference device, showed a good performance but it was less efficient and the replicates were not highly consistent. These three samplers had obtained high value in several diversity indexes, were clustered together in beta diversity analysis and shared the highest number of families while very few OTUs showed significant differences (Figure 2-4). Therefore, we can consider them as suitable for capturing the air diversity, at least regarding bacteria, fungi and plants. However, Burkard and SAS use vaseline for the capture, which is convenient to avoid desiccation, but it represents a problem to extract intact microorganisms. Our attempts to extract viral particles from the Burkard devise vaseline were unsuccessful. Therefore, we decided to use PTFE filters for the analysis of viruses from the air using a similar set as that used for the comparative analysis among samplers. Air was sampled continuously for 8 days at 2.5 L/min getting a total volume of 28,8 m³. Viral particles were purified from the filters and viral DNA was extracted as described in Example 1. Viral DNA was then randomly amplified using MDA and sequenced in a MiSeq machine. After quality filtering, reads were assembled into contigs. Then, contigs were filtered to remove any putative cellular sequence before taxonomic classification. We have also included the data from Whon and Coworkers to compare our results. Most of the reads were classified as viral and only a small fraction was discarded as being putatively from cellular origin (Figure 5A). Of these, most of the contigs were classified as ssDNA viruses (Figure 5B). However, in our data *Genomoviridae* seemed to be a dominant group. *Genomoviridae* is a recently described family of viruses that were found to putatively infect plant pathogenic fungi, although the hosts of these viruses remain largely unknown. Moreover, we could detect one *Genomoviridae* complete circular genome (Figure 5C). Several other contigs were also classified as belonging to *Genomoviridae* but were not assembled completely (data not shown). Additionally, we were able to detect other complete circular genomes belonging to *Geminiviridae* and *Circoviridae* families and one similar to an unclassified ssDNA viral sequence (similar to Pacific flying fox faeces associated circular DNA virus-12, KT732830.1) containing a viral Rep gene (Figure 5C).

### Detection of SARS-CoV2

For experiment 1 and 2, default setting were applied for the analysis. Only cycle threshold (Ct) below 40 with "S" shape were considered as valid. No template controls (NTC) and positive controls were included in all runs. In the table below, Ct for all three genes (E, ORF-1ab, N) in both experiments are shown in table 5.

**Table 5. Location of devices: D1, neonates room; D2, medical doctors room; D3, intensive care unit 1; D4 pediatric emergencies; D5 intensive care unit 2. ND: Not determined; "-": Ct > 40 or not detected.**

| | | **Experiment 1** | | | **Experiment 2** | | |
|---|---|---|---|---|---|---|---|
| **Device** | **Replicate** | **E** | **ORF-1 ab** | **N** | **E** | **ORF-1ab** | **N** |
| D1 | 1 | - | - | 38,9 | 35,2 | 32,7 | 34,8 |
| | | - | | | 36,1 | | |
| | | - | | | 34,2 | | |
| | 2 | - | - | - | 35,1 | - | 36,3 |
| | | - | | | - | | |
| | | - | | | - | | |
| D2 | 1 | - | 35,3 | 37,0 | - | 32,0 | 34,1 |
| | | - | | | 35,3 | | |
| | | - | | | - | | |
| | 2 | 35,3 | 33,8 | 35,7 | - | 38,4 | 38,5 |
| | | - | | | - | | |
| | | 36,3 | | | - | | |
| D3 | 1 | - | - | - | - | - | - |
| | | - | | | - | | |
| | | - | | | - | | |
| | 2 | - | 36,1 | - | - | 32,8 | 36,1 |
| | | - | | | - | | |
| | | - | | | - | | |
| D4 | 1 | ND | ND | ND | 35,7 | 34,9 | 35,3 |
| | | ND | | | - | | |
| | | ND | | | - | | |
| | 2 | ND | ND | ND | - | 33,5 | 34,9 |
| | | ND | | | - | | |
| | | ND | | | - | | |
| D5 | 1 | 36,3 | - | - | - | - | 37,3 |
| | | - | | | - | | |
| | | - | | | - | | |
| | 2 | 35,9 | 35,0 | 36,9 | - | - | - |
| | | 36,8 | | | 35,3 | | |
| | | 36,1 | | | - | | |

### 3. Conclusions.

Here we have presented a comparative analysis of different air samplers. Our first choice was a shotgun approach to obtain a global view of all organisms present in the air. In our experimental setting, plants seemed to dominate regardless of the sampling method used (Figure 1). It is clear that pollen grains are an important component of the airbiota, and, as plants have large genomes, they should be an abundant group in shotgun reads. However, very few complete plant genomes are present in databases, which could introduce an important bias in reads analysis, being underestimated. In our data, *Pinus,* with one of the longest and best studied plant genomes, was the most abundant taxa in almost all samples.

It is interesting to identify the differences in the plant taxonomic profile detected using metataxonomic and shotgun approaches. *Fagales* were the most abundant taxa in the ITS2 profile (-50% on average), while *Pinales* dominated shotgun reads (-80%, Figure 1). This could be partially explained by the different nature of both approaches. For metataxonomic, only a marker gene is amplified regardless the length of the genomes of the species analysed. By contrast, the shotgun approach is clearly influenced by the length of the genomes. Nevertheless, we can normalize the sequencing reads assigned to each taxa by genome length, when available, to be able to compare the data from both approaches. Although, the shotgun sequencing reads corrected by the median genome length results in an important reduction of *Pinales* abundance, from -93% to -30% (only considering plant orders with at least one available genome), the normalized taxonomic profiles were clearly different from that obtained using the metataxonomic approach. These differences could be explained by the lack of information in databases. Plant genomes are scarce in databases, and therefore many shotgun sequences/contigs cannot be properly classified (only 60% of contigs could be classified corresponding to 33% of the sequencing reads). It is also possible that some reads assigned as *Pinus* actually belong to other plants not yet sequenced, and therefore *Pinus* abundance could be also over-estimated to some extent. By contrast, amplicon databases are more curated with a high diversity of sequences available that allow a better classification. It is important to take into account that the most frequently used marker genes are usually multi-copy and their exact number of copies is unknown for many species, and this could also introduce a bias in targeted metagenomic approaches. Nevertheless, beta diversity analysis of shotgun reads, although dominated by plant reads, showed a similar pattern to that observed in targeted metagenomics, being most of the samplers clustered together, except the Impinger (11) and one of the Burkard replicates (B1) (Figures 2-4). These two samples were randomly amplified for 3.5 h while all other samples were only amplified for 2.5 h. MDA is known to introduce biases especially when very small input DNA is used. This could explain the different behavior of samples I1 and B1 in shotgun analysis. However, a similar result was observed in these samples using a targeted metagenomic approach were no MDA was used, and therefore we can consider that these differences are most likely to be real than a bias introduced by MDA amplification.

Nevertheless, using a targeted metagenomic approach we were able to compare different samplers avoiding the dominance of sequencing reads from plant genomes observed by shotgun analysis. In our analysis of the outdoor air, samplers had a low relative influence on the capture of biodiversity, showing most of them a similar taxonomic profile, although some important differences were observed (Figures 1-4). Impingers were almost discarded due to a contamination of bacteria, most likely produced by the growing of bacteria in the buffered solution used. The growing of bacteria could be partially avoided by covering the bottles with aluminum foil to reduce sun incidence, but other non-photosynthetic bacteria would also be able to grow. Impingers are generally used for very short sampling times (-30 min) which reduce the possibility of bacterial growth, and therefore, we can discard this type of samplers when longer sampling times are required. GFC filter sandwiches (Nanoparticles) showed great differences between replicates and among all others samplers. These in house build filters were included to test whether iron coated nanoparticles could increase viral capture, however, they showed great differences when bacteria, fungi or plants are analyzed. As mentioned before, different air flows could be responsible for the differences between replicates, but, in spite of having a similar flow rate than other samplers, the second replicate (N2) also showed important differences in the results. Burkard Multival and BioPump also showed some important differences regarding all other samplers. BM, although showed a similar bacterial profile to most other samplers, did not capture any *Bacillales,* an abundant group (Figure 2). *Agaricales* was also absent from BM profile in fungi analysis, and a great decrease in *Capnodiales* was observed, which produced a more even distribution of other capture taxas (Figure 3). This even distribution caused an increase of the diversity indexes that relays on frequencies (Shannon and Simpson). By contrast, the BM plant profile was similar to that from all other samples (Figure 4), showing high diversity values. BP, although similar in bacteria or fungi analysis, showed a very different plant profile, with an important reduction in *Fagales* an important increase in *Pinales* capture (Figure 4). Similar to BM, these differences in plant BP profile had produced an increase in Shannon and Simpson indexes due to a more even taxa distribution. Unfortunately, no replicates of BM or BP were availables to determine if these differences could be caused by experimental errors or by the capture mechanism by themselves. In this sense, Burkard (B1 and B2) showed great differences between replicates in all groups. B1 showed a different profile in all analysis and was far from the main cluster in all beta diversity analysis. Differences in airflow rates during sampling could explain these capture deficiencies in B1 regarding B2. Airflows were checked at the beginning and at the end of every sampling day, and no differences were observed. However, we cannot discard fluctuations during the sampling. We have observed previously that variation on Burkard airflows produce differences in the recovered DNA and a greater dispersion of the data, especially in bacteria, and therefore, especial care should be taken to control Burkard airflow to reduce this phenomenon. By contrast, PTFE filters and SAS sampler showed a good correlation at profile level in all analysis, even with small airflow differences in PTFE samples (Table 1). These two samplers showed very few differences between them and, followed by the Burkard device, have obtained the best results regarding diversity indexes, number of shared families and a reduced number of OTUs with significant differences in their abundances (Figures 2-4). Therefore, we can conclude that the PTFE and SAS samplers are equally suited to monitor bacteria, fungi and plants on outdoor air, followed by the Burkard device. However, the cost and the portability of the different samplers are important factors to consider them. Burkard is a heavy and fixed device with a high cost, which reduces the possibilities of using this kind of devices to only a limited number of places. Burkard Multival and BioPump are also very expensive and, although BP is portable, its high cost also reduces the possibilities of sampling many places simultaneously. Impingers are the cheapest devices but as mentioned care should be taken when long sampling times are used to reduce bacterial growth, for example using azide. SAS is also expensive, but two replicates can be sampled at the same time which makes this sampler more flexible, but again sampling several localization simultaneously could be prohibitive using this device. Finally, filter samplers, which are mainly a vacuum pump with a filter holder, are cheaper than others, replicates can be sampled using two or more filter holders, and several localization can be studied simultaneously using several devices with a reduce cost.

Apart from the cost, one of the major challenges for study airborne microorganisms is the amount of biomass and therefore the amount of DNA that can be obtained. This could be partially solved by the used of random amplification methods such as MDA or Sequence-Independent Single-Primer Amplification (SISPA). However, as shown here, when a shotgun approach is used, it is important to consider the presence of pollen that can dominate most of the reads, although most of them could not be classified due to database deficiencies. In this sense, targeted metagenomics could be a good solution. However, viruses cannot be studied following this targeted approach. Viruses do not have any common marker gene to study them, and only a shotgun approach can be used. Using such approach, in spite of the dominance of plant reads, we were able to detect viral contigs, being some of them complete genomes. Moreover, we were able to detect and describe a new viral genome (*Pinus nigra virus 1*) belonging to a new putative *Caulimoviridae* genus infecting gymnosperms. By contrast, the circo-like contig detected in B1 sample that was 99% identical to MG023129.1, a viral genome detected on marine isopods, could represent a contamination of the silica used in the DNA extraction kit columns as it was previously reported.

Additionally, viruses can have DNA or RNA genomes and analysing only DNA will result in a biased view of viral diversity. Moreover, most human infecting viruses are RNA viruses such as *Influenza* or *Enterovirus* (*Rhinovirus*). Therefore, for environments with very different organisms and a variety of genome lengths, the best option to study viruses is the purification of virus particles before sequencing. However, this is not an easy task for most samplers. For example, SAS and Burkard use vaseline layers to capture the airborne particles and the purification of viruses or cells from this substance, by using chloroform or other organic solvent, can destroy most of cellular organisms and some viruses. As we show here, PTFE filters represent the best option for viral metagenomics because of the easy to extract cellular organism and virus particles by shaking or sonication of the filters in a buffer.

In conclusion, we have tested several sampling devices, using different capturing mechanisms, for the analysis of the airborne biological community in order to establish a standardized method. First, we tried using a shotgun sequencing approach, a method that would allow the sequencing of all microorganisms present in the samples. However, we observed that this approach is not the most convenient when the community is composed by organisms with very different genomes sizes. In our experiments, pollen grains from *Pinus,* which have a very large genome (22 Gb), have monopolized most of the sequencing reads and only a small fraction of the reads belonged to other taxonomic groups. A deeper sequencing could solve partially this problem, although the sequencing cost could also make the use of this approach prohibitive. Therefore, community diversity should be considered before using a shotgun sequencing approach. By contrast, amplicon sequencing, a simpler and cheaper method, allowed us to compare the communities captured by the different sampling devices, despite the genome size diversity. Using this approach, we showed that PTFE filters and SAS are the most suitable samplers for the analysis of bacteria, fungi spores and pollen from plants, with very little differences between them. Other samplers, such as Burkard, also showed a good performance, but the consistency between replicates was poor compared to SAS and PTFE, which is crucial in comparative studies. Additionally, taking into account other factors such as cost and portability, PTFE filters were the most convenient device. Finally, we have demonstrated that PTFE filters allow the extraction of the viral fraction for viral metagenomics, which allows the analysis of the whole airborne biological community.

## Claims

1. Device for capturing air biological particles comprising:
• a substrate configured to capture particles from flowing air thereon using at least one captor; and
• at least one processor operable to enable the air to flow through or over the substrate, thereby causing the particles in the air to be captured by the substrate;
wherein the substrate and the at least one processor are communicatively coupled.

2. The device according to claim 1, wherein the substrate comprises at least one filter acting as the at least one captor.

3. The device according to claim 2, wherein the at least one filter is a polytetrafluorethylene (PTFE) filter, preferably the polytetrafluoroethylene (PTFE) filter is a synthetic fluoropolymer of tetrafluoroethylene filter.

4. The device according to claim 3, wherein the polytetrafluorethylene (PTFE) filter has a nominal pore size between 0.2µm and 5 µm, preferably 1 µm or 5 µm, more preferably 1 µm.

5. The device according to any one of preceding claims, further comprising an air pump operable to increase or decrease flow of air, preferably the air pump is a vacuum pump,
wherein, to capture the particles into the substrate, the at least one processor is operable to:
cause the air pump to increase or decrease the flow of air containing the particles toward the substrate.

6. A system for detecting and identifying organisms present in the air comprising the device of any one of the preceding claims.

7. The system of claim 6 further comprising:
• centrifugal means configured to remove cellular organisms from the substrate, and
• buffering means configured to house the substrate, and
• preferably further comprising a filtration means configured to filtrate the supernatants containing the virus particles, wherein the centrifugal means are also configured to concentrate the supernatants.

8. The system of claim 7 further comprising:
• nuclease treatment means configured to remove all non-encapsidated DNA or RNA.

9. The system of claim 8 further comprising:
• gene amplification means for all or specific viral genomes and sequencing means for identification or detection of specific virus or collective viruses

10. Use of the device according to any one of claims 1 to 5 or the system according to any one of claims 6 to 9 for collecting, detecting and identifying organisms present in air, preferably the organisms are air pathogens.

11. The use according to claim 10, wherein the organisms are selected from the list consisting of: viruses, plants, bacteria, pollen and fungi, preferably the organisms are viruses, more preferably the virus is SARS-CoV2.

12. A method for collecting, detecting and identifying organisms present in the air, preferably the organisms are air pathogens, wherein said method comprises the steps of:
a. sampling an air sample with the device according to any one of claims 1 to 5,
b. extracting the genetic material comprised in the device after step (a),
c. randomly amplifying the genetic material extracted in step (b), and
d. sequencing the genetic material amplified in step (c).

13. The method according to claim 12, wherein the organisms are selected from the list consisting of: viruses, plants, bacteria, pollen and fungi, preferably the organisms are viruses, more preferably the virus is SARS-CoV2.

14. The method according to any of claims 12 or 13, which comprises an additional step (a'), between steps (a) and (b), in which cellular organisms are removed.

15. The method according to any one of claims 12 to 14, wherein the amplification of step (c) is performed by multiple displacement amplification (MDA) or PCR.


SEQUENCE LISTING

<110>  Consejo Superior de Investigaciones Cientificas (CSIC)

<120>  DEVICE AND METHOD FOR CAPTURING AND ANALYSING AIRBORNE ORGANISMS

<130>  EP1641.1564

<160>  9

<170>  PatentIn version 3.5

<210>  1
<211>  17
<212>  DNA
<213>  Artificial Sequence

<220>
<223>  Primer BacF



<220>
<221>  misc_feature
<222>  (9)..(9)
<223>  n is a, c, g, or t

<400>  1
cctacgggng gcwgcag                                                    17


<210>  2
<211>  21
<212>  DNA
<213>  Artificial Sequence

<220>
<223>  Primer BacR

<400>  2
gactachvgg gtatctaatc c                                               21


<210>  3
<211>  21
<212>  DNA
<213>  Artificial Sequence

<220>
<223>  Primer ITS86F

<400>  3
gtgaatcatc gaatctttga a                                               21


<210>  4
<211>  20
<212>  DNA
<213>  Artificial Sequence

<220>
<223>  Primer ITS4

<400>  4

tcctccgctt attgatatgc                                                    20


<210>  5
<211>  19
<212>  DNA
<213>  Artificial Sequence

<220>
<223>  Primer ITSD-For

<400>  5
ygactctcgg caacggata                                                     19


<210>  6
<211>  20
<212>  DNA
<213>  Artificial Sequence

<220>
<223>  Primer ITS4-Rev

<400>  6
tcctccgctt attgatatgc                                                    20


<210>  7
<211>  26
<212>  DNA
<213>  Artificial Sequence

<220>
<223>  Primer E_Sarbeco_F1

<400>  7
acaggtacgt taatagttaa tagcgt                                             26


<210>  8
<211>  22
<212>  DNA
<213>  Artificial Sequence

<220>
<223>  Primer E_Sarbeco_R2

<400>  8
atattgcagc agtacgcaca ca                                                 22


<210>  9
<211>  26
<212>  DNA
<213>  Artificial Sequence

<220>
<223>  Primer E_Sarbeco_P1

<400>  9
acactagcca tccttactgc gcttcg                                             26